# EUROPEAN PATENT SPECIFICATION

(11) **EP 0 525 659 B1**
(45) Date of publication and mention of the grant of the patent: **02.04.1997**
(21) Application number: 92112647.0
(22) Date of filing: 24.07.1992
(51) Int. Cl.: C07C 31/24, C07C 29/78, C12P 7/18

(54) **Process for preparing erythritol crystals**
Verfahren zur Herstellung von Erythritolkristallen
Procédé de préparation de cristaux d'érythritol

(30) Priority: 26.07.1991 JP 187804/91; 08.10.1991 JP 260871/91
(43) Date of publication of application: 03.02.1993
(73) Proprietor: Mitsubishi Chemical Corporation, Chiyoda-ku Tokyo (JP); Nikken Chemicals Company, Limited, Tokyo 104 (JP)
(72) Inventor: Maeda, Toshihiro, Zama-shi, Kanagawa (JP); Shida, Makoto, Ebina-shi, Kanagawa (JP); Ohshima, Yoshikazu, Kitakyushu-shi, Fukuoka (JP); Yamada, Tetsuo, Onga-gun, Fukuoka (JP); Fujimura, Kouji, Shimonoseki-shi, Yamaguchi (JP)
(74) Representative: VOSSIUS & PARTNER

(56) References cited:
- EP-A- 0 262 463
- EP-A- 0 327 016
- LEBENSMITTEL-WISSENSCHAFT UND TECHNOLOGIE, vol. 10, no. 1, 1977, Zürich, CH, pages 36 - 39, T. PYSSALO et al, "Odour thresholds of the major volatiles identified in cloudberry and arctic bramble"

## Description

This invention relates to a process for preparing erythritol crystals by fermentation. More particularly, it relates to a process for preparing erythritol crystals suitable for food which has tasteful sweetness without an irritating chemical smell and also has excellent crystal properties.

Erythritol is a tetrahydric sugar alcohol having a molecular formula C₄H₁₀O₄ and a molecular weight of 122. Erythritol obtained by fermentation has a meso form as represented by structural formula: 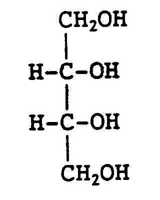 The terminology "erythritol" as used herein means mesoerythritol.

Erythritol is about 0.8 times as sweet as sucrose and is attracting attention as a low-calory sweetener. It is generally produced by aerobically culturing yeast like fungi, e.g., Aureobasidium sp. SN-G42 (U.S. Patent 5,036,011, JP-A-3-43091; the term "JP-A" as used herein means an "unexamined published Japanese patent application"), Moniliella tomentusa var. polinis (European Patents 136,803, 136,804, and 136,805), or Candida lypolytica (U.S. Patent 3,756,917, and JP-B-47-41549; the term "JP-B" as used herein means an "examined published Japanese patent application"), in an aqueous medium containing glucose. The resulting culture, from which the microbial cells have been removed, is usually purified by an appropriate combination of activated carbon treatment, ion-exchange resin treatment, chromatographic separation, and the like, concentrated, and crystallized, followed by separation and drying of the resulting crystals.

The erythritol crystals obtained by the conventional fermentation processes has a disadvantage of smell. That is, a smelling component by-produced in a trace amount during fermentation cannot be completely removed by purification steps and still remains in the crystallized product to give off a chemical smell. It has been difficult to obtain odorless erythritol crystals having tasteful sweetness.

Further, the conventional fermentation erythritol crystals generally show a tendency to agglomeration. The agglomerated crystals generally have poor mechanical strength and are liable to be broken and powdered and, on the other hand, liable to cake.

It is the object of the present invention to provide crystalline erythritol free from chemical smell and having very tasteful sweetness.

This object could be achieved on the basis of the finding that the smell of erythritol crystals depends on the concentration of acetoin which is by-produced during fermentation and that erythritol crystals which are free from a chemical smell and has very tasteful sweetness can be obtained by controlling an acetoin concentration of an erythritol-containing liquid to be crystallized within a specific range.

The present invention relates to a process for preparing erythritol crystals comprising crystallizing an erythritolcontaining liquid obtained by fermentation, wherein the acetoin concentration of the erythritol-containing liquid is controlled to 20 ppm by weight or less.

In the present invention, erythritol-containing liquid can be obtained by fermentation in a conventional manner, for example, by the method described in U.S. Patent 5,036,011 using Aureobasidium sp. SN-G42. The microorganism Aureobasidium sp. SN-G42 strain is a mutant induced by irradiating a microorganism Aureobasidium sp. SN-124A strain (parent strain) with ultraviolet rays and treating with a mutagenic agent, e.g., N-methyl-N'-nitro-N'-nitrosoguanidine, and it has been deposited with Fermentation Research Institution of Agency of Industrial Science and Technology under FERM P-8940 (FERM BP-1430).

The Aureobasidium sp. SN-G42 strain is preferably cultured under aerobic conditions on a liquid culture medium containing an assimilable carbon source, an assimirable nitrogen source, inorganic salts and the like.

For the carbon source of the liquid culture media, use may be made of fermentable sugars such as glucose, fructose and sucrose. A sugar concentration is generally 10 to 55 %, preferably 20 to 50 %.

As the nitrogen sources, nitrogen compounds available by microorganisms are used such as, for instance, yeast extract, peptone, malt extract, casamino acids, corn steep liquor, ammonium sulfate and urea. These nitrogen sources are preferably used in an amount of 0.5 to 3.0 % for yeast extract, and 1.5 to 10 % for corn steep liquor.

The inorganic salts used include salts such as ferrous sulfate, potassium chloride, sodium chloride, dipotassium hydrogen phosphate, and calcium hydroxide, and are preferably used in an amount not more than 0.1 %.

It is to be noted that in addition to these carbon sources, nitrogen sources and inorganic salts, various organics and inorganics required for the growth of microorganisms or conventionally used defoamers or the like may be added.

For culture, the cells of SN-G42 strain may be inoculated directly on the liquid culture media having the aforesaid composition, or a separate seed culture solution obtained by pre-culture may be inoculated thereon. Such a seed culture solution is prepared by the inocuation of a loopfull of yeast-like slant-cultured in the conventional manner on a liquid culture medium of pH 4 to 6 containing 33.5 % of glucose and 4.5 % of corn steep liquor, followed by culture at 34° to 36°C for 2 to 4 days.

Culture is carried out in a temperature range in which microorganisms can grow, i.e., at 30° to 38°C., preferably 35° to 37°C.

The culture media are adjusted to pH 4 to 9, preferably 4 to 7.

The culture period varies depending upon the type of the media used and the concentration of sugar that provides the carbon source, but is usually about 4 to 8 days.

The culture thus obtained contains erythritol and is subjected to the process of the present invention.

The process according to the present invention basically comprises the steps of removal of microbial cells from a culture, purification, concentration, and crystallization as in the conventional technique. It should be noted, however, that mere following of the conventional processes fails to solve the problem of smell for an extended period of time. It is necessary to manipulate production conditions so as to enable sufficient reduction of acetoin by-produced during erythritol fermentation as hereinafter described.

Acetoin, represented by formula CH₃CH(OH)COCH₃, has a melting point of 15°C and a boiling point of 148°C and gives off an irritating chemical smell. It is known as a by-product accompanying production of fermentation products. For example, beer, sake, wine, cider, or vinegar as a final product is known to contain acetoin in a concentration of 18 to 26 ppm by weight, 4 to 30 ppm by weight, 20 ppm by weight or less, 300 to 400 ppm by weight, or 800 ppm by weight or less, respectively. As far as these fermentation products are concerned, the irritating chemical smell of acetoin has given rise to no problem because the products are liquids comprising many components and the acetoin present therein is in harmony with other odor components.

The acetoin concentration in the erythritol fermentation liquid usually ranges from 25 to 300 ppm by weight, though somewhat varying depending on culturing conditions. Unlike the above-mentioned other fermentation products, an erythritol crystal has a purity reaching almost 100% by thorough purification prior to crystallization, with an acetoin concentration being not more than 1 ppm, which is below the detection limit in gas chromatographic analysis. Therefore, it has never been considered that the irritating smell of an erythritol crystal should be ascribable to acetoin. Since an erythritol crystal has high purity and contains no other odor components, it appears that the smell of acetoin even in such a trace amount as being below the detection limit would be emphasized and become perceptible as an irritating chemical smell.

In order to reduce the acetoin in the final erythritol product to an imperceptible degree, the acetoin concentration in the erythritol-containing liquid in a crystallizer should be controlled to 20 ppm by weight or less, preferably between 1 and 20 ppm by weight, and more preferably between 5 and 15 ppm by weight. Considering that an acetoin concentration of less than 1 ppm by weight is imperceptible by human nose and that reduction of acetoin concentration below this level would incur high cost, the acetoin concentration in the liquid to be crystallized usually does not need to be reduced below 1 ppm.

Achievement of acetoin control within the above-recited range requires careful control of conditions in the purification operations following culturing up to crystallization. In order to effectively remove acetoin by purification and to prevent incorporation of acetoin into crystals during crystallization, the most effective means to be adopted is evaporation separation. Acetoin can be evaporated off together with water by concentration. The evaporation loss of acetoin can be increased by repeating dilution and concentration operations with addition of water. Nevertheless, mere addition of fresh water to a concentrate is impractical. It is desirable to concentrate an erythritol-containing liquid which is sufficiently diluted with water used in a purification step, etc. as described below.

Wet microbial cells separated from the culture are washed usually one to three times with a sufficient amount of water (usually half to double the volume of the wet cells), and the erythritol contained in the washings is recovered as much as possible. The washing from which erythritol has been recovered is then added to the culture from which microbial cells have been removed thereby to obtain a diluted erythritol-containing liquid. Likewise, the washings resulting from purification operations, such as ion-exchange resin treatment and activated carbon treatment, are also used for dilution of the erythritol-containing liquid.

When the culture liquid is purified only by ion-exchange resin treatment and activated carbon treatment, the resulting erythritol-containing liquid still contains fermentation by-products, such as glycerin, or other impurities, such as oligosaccharides, resulting in a reduction in crystallizing efficiency and a reduction in purity. It is preferred, therefore, to subject the culture liquid to chromatographic separation as described, e.g., in U.S. Patent 4,906,569. The chromatographic separation can be carried out by concentrating a culture liquid and passing the concentrate through a chromato-column packed with an alkali metal type or ammonium type strongly acidic cation exchange resin. Since the erythritol-containing fraction is diluted through this chromatographic separation, it should be re-concentrated before crystallization. Such a double-stage concentration operation is particularly effective for evaporation of acetoin.

After a concentrate of an erythritol-containing liquid is crystallized, the wet crystals formed are separated from the mother liquor usually by centrifugation. The wet crystals are then washed with a sufficient amount of water (usually 0.2 to 1.5 times the weight of the crystals). Because both the mother liquor and the washing are expected to contain a considerable amount of erythritol, at least a part of them is recycled to any of the steps preceding crystallization to thereby dilute the erythritol-containing liquid to be concentrated.

Since the mother liquor separated after crystallization contains acetoin and other impurities in a concentrated state, a part of the mother liquor may be withdrawn from the production line to remove the impurities. The acetoin concentration may be adjusted also by this withdrawal. Withdrawal of the mother liquor more than necessary leads to a reduction in recovery of the erythritol crystals. It is preferable to first subject the culture liquid to the above-described chromatographic separation to previously remove the most of the impurities thereby to minimize the amount of the impurities accumulated in the crystallizing mother liquor.

Where the mother liquor and/or the washing is returned to the step preceding the crystallization step, they, especially the former, may be directly returned to the concentration step but are preferably returned to any of the step of chromatographic separation, the step of activated carbon treatment, and the step of ion-exchange resin treatment to conduct re-purification. The re-purification, particularly the ion-exchange resin treatment, is followed by concentration and crystallization to obtain erythritol crystals which are free from an irritating chemical smell and has tasteful sweetness resembling that of sucrose. The ion-exchange resin treatment of the mother liquor after crystallization may be effected in the same apparatus as used for the treatment of the culture liquid with a time lag or in a separately provided apparatus.

The thus diluted erythritol-containing liquid is concentrated to a concentration suited for crystallization, during which acetoin is removed from the liquid by evaporation. Concentration is usually carried out at a temperature of from 50° to 100°C under atmospheric pressure or under reduced pressure. The apparatus to be used for concentration includes an ordinary multiple effect evaporator, a Kestoner evaporator, and a thin film type evaporator.

It has been believed basically preferable for reducing the acetoin concentration and increasing the crystallizing efficiency that the erythritol-containing liquid be concentrated as highly as possible. For example, from the fact that erythritol has a water solubility of about 3 times by weight at 80°C, the erythritol-containing liquid has hitherto been concentrated to have a weight 2 to 3 times that of water prior to crystallization. However, under such a concentration condition, the precipitated erythritol crystals tend to become agglomerated crystals, which have poor mechanical strength and are easily powdered and, on the other hand, are liable to cake. In addition, the resulting slurry containing a large amount of the crystals is sometimes difficult to run through a pipeline. Taking the crystallizing efficiency and crystal state into consideration, a preferred erythritol concentration in the erythritol-containing liquid in a crystallizer is from 0.4 to 1.5 times the weight of water, i.e., from about 30 to 60% by weight, and particularly from 0.7 to 1.2 times the weight of water, i.e., from 40 to 55% by weight.

Crystallization may be carried out in either a batch system or a continuous system. In the latter case, the concentration of the erythritol inclusive of the precipitated crystals is maintained within the above range based on the total water content in a crystallizer.

Crystallization is usually performed by heating the erythritol-containing liquid to a temperature of 50°C or higher, and preferably 60° to 90°C, followed by gradual cooling to 20°C or lower, and preferably 15°C or lower. While taking time in cooling is preferable, the rate of cooling may be somewhat increased by addition of seed crystals without adversely affecting the crystal properties. The amount of seed crystals to be added usually ranges from 0.005 to 0.5% by weight, and preferably from 0.01 to 0.2% by weight, based on the erythritol in the liquid. If the amount of the seed crystals is too small, the increase in cooling rate causes a failure of obtaining a desired single crystal in a stable manner. If it is excessive, the resulting crystals would have a reduced size. The erythritol seed crystals preferably have relatively small particle size, e.g., from 50 to 100 µm. The time of addition of the seed crystals is important for stable production of erythritol crystals. A suitable stage of addition varies depending on the erythritol concentration and the like but is, in general, before precipitation of a crystal and at the time when the liquid temperature decreases to a range of from 15 to 50°C, and preferably from 20 to 45°C.

Under the above-described crystallizing conditions, there are obtained erythritol crystals having a large size (usually 500 to 700 µm, the same as the size of ordinary granulated sugar), having high mechanical strength, and having crystal properties similar to granulated sugar. Where the resulting erythritol crystals are mixed with granulated sugar to be served as table sugar, the mixed product looks uniform because of the similarity in particle size.

The present invention is now illustrated in greater detail with reference to Examples, but it should be understood that the present invention is not deemed to be limited thereto. All the percents and ppm are by weight unless otherwise indicated.

### EXAMPLE 1

### 1) Culturing:

In each of three 3 ℓ-volume Erlenmeyer flasks was put 0.6 ℓ of a sterilized seed culture medium containing 300 g/ℓ of purified glucose, 2.5 g/ℓ of yeast extract, 2.5 g/ℓ of malt extract, and 5 g/ℓ of polypeptone. The medium was inoculated with Aureobasidium sp. SN-G 42 and shake-cultured at 30°C for 96 hours to prepare a preculture.

In a sterilized 50 ℓ jar fermentor was charged a sterilized aqueous solution containing 11.16 kg of purified glucose (purity: 95%) and 1.87 kg of sterilized corn steep liquor (hereinafter abbreviated as CSL), and 1.62 ℓ of the above obtained preculture was added thereto. After the pH was adjusted to 4.2, the system was subjected to culturing. The system at the commencement of culturing had a volume of 26.7 ℓ, comprising 397 g/ℓ of glucose and 70 g/ℓ of CSL. Culturing was carried out at 35°C for 96 hours at a stirring speed of 600 rpm and an air feed rate of 0.5 vvm. The culture at the end of the culturing had a volume of 24.2 ℓ, comprising 214.3 g/ℓ of erythritol, 17.5 g/ℓ of glycerin, 67.7 mg/ℓ of acetoin, and 10.8% by volume of microbial cells. Half of the resulting culture (12.1 ℓ) was purified as follows.

### 2) Separation of Microbial Cells:

The culture (12.1 ℓ) was separated into wet microbial cells and a supernatant liquor by means of a de Laval type centrifuge. To the separated wet microbial cells was added water of equal volume (2.05 ℓ), followed by stirring. The slurry was again separated into wet microbial cells and a supernatant liquor (washing) by means of the same centrifuge. This washing operation was repeated once more under the same conditions. All the washings were combined with the supernatant obtained by the first centrifugal separation, reaching a total of 14.2 ℓ.

### 3) Ion-Exchange Resin Treatment:

The liquid (14.2 ℓ) was demineralized by passing through an ion-exchanger composed of a column packed with 1.5 ℓ of a strongly acidic cation-exchange resin ("Diaion SKIB, H type" produced by Mitsubishi Kasei Corp.), a column packed with 1.5 ℓ of a weakly basic anion-exchange resin ("Diaion WA 30, OH type" produced by Mitsubishi Kasei Corp.), and a mixed bed column packed with a mixture of 0.5 ℓ of a strongly acidic cation-exchange resin ("Diaion SKIB, H type") and 1.0 ℓ of a strongly basic anion-exchange resin ("Diaion PA 408, OH type" produced by Mitsubishi Kasei Corp.), all having a diameter of 50 mm and a packing length of 765 mm, connected in series. After all the liquid was introduced into the columns at a space velocity of 2 hr⁻¹, the columns were swept with water at the same space velocity. Collection of the effluent was started from the time point when 2.7 ℓ of the liquid had been fed, at which diluted erythritol began to effuse from the mixed bed column, up to the end of effusion of erythritol. The total amount of the effluent collected was 18.2 ℓ (19.4 kg).

### 4) Treatment with Activated Carbon:

To the collected liquid was added 31 g of activated carbon powder, and the mixture was thoroughly stirred and filtered. The separated activated carbon was washed with 93 g of water, and the washing was added to the activated carbon-treated liquid.

The erythritol recovery attained at this point was as high as 98.3% by virtue of the thorough washing of the microbial cells, sweeping of the ion-exchange columns with water, and washing of the activated carbon. Because of the use of a large quantity of water in the washing and column sweeping operations, the amount of the liquid increased to 19.4 kg, while the erythritol concentration decreased to 13.1%. Then, the activated carbon-treated liquid was concentrated to 4.31 kg under the conditions of 65°C and 150 mmHg. The concentrate contained 59.5% of erythritol and 16.5 ppm of acetoin. The rate of acetoin removal in the concentration step was 91%.

### 5) Crystallization:

The concentrate was slowly cooled in a crystallizer from 70°C to 20°C over a period of 8 hours, and the precipitated crystals were collected by filtration. The wet crystals were washed with 1.01 kg (half the weight of the wet crystals) of cold water at 5°C and then dried under reduced pressure to obtain 1.74 kg of erythritol crystals. The resulting crystals had no irritating smell of chemicals.

The crystallizing mother liquor and the washing were combined to amount to 3.58 kg, having an erythritol concentration of 23.0% and an acetoin concentration of 19.9 ppm.

### COMPARATIVE EXAMPLE 1

The remainder of the culture obtained in Example 1-(1) (12.1 ℓ) was purified as follows.

### 1) Separation of Microbial Cells:

The culture (12.1 ℓ) was separated into wet microbial cells and a supernatant liquor by means of a de Laval type centrifuge. The wet microbial wells were discarded without being washing with water. The supernatant liquor (not diluted) was 10.1 ℓ.

### 2) Ion-Exchange Resin Treatment:

The liquid (10.1 ℓ) was demineralized by passing through the same ion-exchanger as used in Example 1-(3) at a space velocity of 2 hr⁻¹. After all the liquid was introduced into the columns, the columns were swept with water at the same space velocity. In order to prevent the erythritol-containing liquid from being diluted, collection of the effluent was started at the time point when the erythritol concentration of the effluent from the mixed bed column increased to half of that of the liquid fed, and collection was stopped at the time point when the erythritol concentration of the effluent was reduced to half of that of the liquid fed in order to avoiding excessive dilution.

### 3) Activated Carbon Treatment:

To the demineralized liquid collected was added 25 g of activated carbon, and the mixture was thoroughly stirred and filtered.

Because dilution with water was avoided during the above treatments with ion-exchange resins and with activated carbon, the resulting filtrate weighed 11.1 kg and had a high erythritol concentration of 18.3%. However, the erythritol recovery at this point was as low as 78.1%.

### 4) Concentration:

The activated carbon-treated liquid was concentrated to 4.22 kg. The concentrate had an erythritol concentration of 48.0% and an acetoin concentration of 33.4 ppm. The rate of acetoin removal during the concentration step was 78%.

### 5) Crystallization:

The concentrate was slowly cooled in a crystallizer from 55°C to 20°C over a period of 8 hours, and the precipitated crystals were collected by filtration. The wet crystals were washed with 614 g (half the weight of the wet crystal) of cold water at 5°C and then dried under reduced pressure to obtain 1.06 kg of erythritol crystals. The resulting crystals had a weak irritating smell of chemicals.

### EXAMPLE 2

### 1) Culturing:

In each of four 3 ℓ-volume Erlenmeyer flasks was put 0.6 ℓ of a sterilized seed culture medium containing 300 g/ℓ of purified glucose, 2.5 g/ℓ of yeast extract, 2.5 g/ℓ of malt extract, and 5 g/ℓ of polypeptone. The medium was inoculated with Aureobasidium sp. SN-G 42 and shake-cultured at 30°C for 96 hours to prepare a preculture.

In a sterilized 50 ℓ jar fermentor was charged a sterilized aqueous solution containing 12.12 kg of purified glucose (purity: 95%) and 1.87 kg of a sterilized CSL, and 1.90 ℓ of the above obtained preculture was added thereto. After the pH was adjusted to 4.2, the system was subjected to culturing. The system at the commencement of culturing had a volume of 30.7 ℓ, comprising 375 g/ℓ of glucose and 61 g/ℓ of CSL. Culturing was carried out at 35°C for 142 hours at a stirring speed of 300 rpm and an air feed rate of 0.25 vvm. At the end of the culturing, the culture had a volume of 23.2 ℓ, comprising 198 g/ℓ of erythritol, 12 g/ℓ of glycerin, 102 mg/ℓ of acetoin, and 15.8% by volume of microbial cells.

The half portion, i.e., 11.6 ℓ, of the resulting culture was purified as follows.

### 2) Separation of Microbial Cells:

The culture (11.6 ℓ) was separated into wet microbial cells and a supernatant liquor by means of a de Laval type centrifuge. To the wet microbial cells thus separated was added water of equal volume (2.96 ℓ), followed by stirring. The mixture was again separated into wet microbial cells and a supernatant liquor (washing) by means of the same centrifuge. This washing operation was repeated once more under the same conditions. All the washings were combined with the supernatant liquor obtained by the first centrifugal separation, reaching a total of 14.6 ℓ.

### 3) Chromatographic Separation:

Prior to chromatography for removing salts and oligosaccharides originated in the purified glucose, the liquid was concentrated to 6.74 kg. The concentrate had an erythritol concentration of 33.3% and an acetoin concentration of 55.1 ppm. The rate of acetoin removal during the concentration step was 68%.

The concentrate was passed through a column (250 mm in diameter; 1170 mm in packing length) packed with 57.4 ℓ of a strongly acidic cation-exchange resin "Diaion UBK 530, Na type" (produced by Mitsubishi Kasei Corp.) at a space velocity of 0.57 hr⁻¹. After all the concentrate was fed, water at 75°C was successively supplied. The effluent from the column bottom up to 34.4 ℓ mainly comprised salts present in the concentrate and was discarded. Since the subsequent effluent contained erythritol, collection of the effluent was then started. Collection was stopped when the effluent collected amounted to 16.1 ℓ, where no erythritol was detected any more.

### 4) Ion-Exchange Resin Treatment:

The liquid (16.1 ℓ) was demineralized by means of an ion-exchanger in the same manner as in Example 1-(3). The total amount of the effluent collected was 20.1 ℓ (21.1 kg).

### 5) Activated Carbon Treatment:

To the collected liquid was added 25 g of powdered activated carbon, and the mixture was thoroughly stirred and filtered. The separated activated carbon was washed with 75 g of water, and the washing was added to the activated carbon-treated liquid.

The erythritol recovery attained at this point was as high as 96.6% by virtue of the thorough washing of the microbial cells, sweeping of the ion-exchange columns with water, and washing of the activated carbon. Because of the use of a large quantity of water in the washing and column sweeping operations, the amount of the liquid increased to 21.1 kg, while the erythritol concentration decreased to 10.5%. Then, the activated carbon-treated liquid was concentrated to 4.05 kg. The concentrate contained 54.7% of erythritol and 7.9 ppm of acetoin. The overall rate of acetoin removal attained by the double-stage concentration was 97%.

### 6) Crystallization:

The concentrate was slowly cooled in a crystallizer from 60°C to 20°C over a period of 8 hours, and the precipitated crystals were collected by filtration. The wet crystals were washed with 754 g (half the weight of the wet crystals) of cold water at 5°C and then dried under reduced pressure to obtain 1.30 kg of erythritol crystals. The resulting crystals had no irritating chemical smell.

The crystallizing mother liquor and the washing were combined to amount to 3.51 kg, having an erythritol concentration of 26.3% and an acetoin concentration of 10.5 ppm.

### COMPARATIVE EXAMPLE 2

The remainder of the culture obtained in Example 2-(1) (11.6 ℓ) was purified as follows.

### 1) Separation of Microbial Cells:

The culture (11.6 ℓ) was separated into wet microbial cells and a supernatant liquor by means of a de Laval type centrifuge. To the wet microbial cells separated was added water of equal volume (2.96 ℓ), followed by stirring. The slurry was again separated into wet microbial cells and a supernatant liquor by means of the same centrifuge. The thus separated supernatant liquor was combined with the previously separated supernatant liquor, reaching a total of 11.6 ℓ.

### 2) Ion-Exchange Resin Treatment:

The liquid (11.6 ℓ) was demineralized by passing through the same ion-exchanger as used in Example 1-(3) at a space velocity of 2 hr⁻¹. After all the liquid was introduced into the columns, the columns were swept with water at the same space velocity. In order to prevent the erythritol-containing liquid from being diluted, collection of the effluent was started at the time point when the erythritol concentration of the effluent from the mixed bed column increased to half of that of the liquid fed, and collection was stopped at the time point when the erythritol concentration of the effluent was reduced to half of that of the liquid fed in order to avoiding excessive dilution.

### 3) Activated Carbon Treatment:

To the collected liquid was added 25 g of activated carbon, and the mixture was thoroughly stirred and filtered.

Because dilution with water was avoided during the above treatments with ion-exchange resins and with activated carbon, the resulting filtrate weighed 12.6 kg and had a high erythritol concentration of 16.0%. However, the erythritol recovery at this point was as low as 87.6%.

### 4) Concentration:

The activated carbon-treated liquid was concentrated to 3.99 kg. The concentrate had an erythritol concentration of 50.4% and an acetoin concentration of 44.4 ppm. The rate of acetoin removal during the concentration step was 83%.

### 5) Crystallization:

The concentrate was slowly cooled in a crystallizer from 58°C to 20°C over a period of 8 hours, and the precipitated crystals were collected by filtration. The wet crystals were washed with 657 g (half the weight of the wet crystals) of cold water at 5°C and then dried under reduced pressure to obtain 1.13 kg of erythritol crystals. The resulting crystals had a strong irritating smell of chemicals.

### EXAMPLE 3

The mixture of the mother liquor and the washing as obtained in Example 1-(5) weighing 3.58 kg was divided into two portions. One portion (1.79 kg) was purified through the following steps (1) to (3), while the other portion was purified through the following steps (1') to (2').

### 1) Ion-Exchange Resin Treatment:

The liquid (1.79 kg) was demineralized by passing through an ion-exchanger composed of a column packed with 0.2 ℓ of a strongly acidic cation-exchange resin ("Diaion SKIB, H type" produced by Mitsubishi Kasei Corp.), a column packed with 0.2 ℓ of a weakly basic anion-exchange resin ("Diaion WA 30, OH type" produced by Mitsubishi Kasei Corp.), and a mixed bed column packed with a mixture of 0.067 ℓ of a strongly acidic cation-exchange resin ("Diaion SKIB, H type") and 0.133 ℓ of a strongly basic anion-exchange resin ("Diaion PA 408, OH type" produced by Mitsubishi Kasei Corp.), all having a diameter of 20 mm and a packing length of 637 mm, connected in series. After all the liquid was introduced into the columns at a space velocity of 2 hr⁻¹, the columns were swept with water at the same space velocity. At the time point when 0.36 ℓ of the liquid had been fed, diluted erythritol began to effuse from the mixed bed column, at which collection of the effluent was started. The total amount of the effluent collected up to the end of effusion of erythritol was 2.33 kg. The thus treated liquid had an erythritol concentration of 17.7%.

### 2) Concentration:

The treated liquid was concentrated to 1.01 kg. The concentrate had an erythritol concentration of 40.7% and an acetoin concentration of 9.3 ppm. The rate of acetoin removal during the concentration step was 74%.

### 3) Crystallization:

The concentrate was slowly cooled in a crystallizer from 57°C to 20°C over a period of 8 hours, and the precipitated crystals were collected by filtration. The wet crystals were washed with 133 g (half the weight of the wet crystals) of cold water at 5°C and then dried under reduced pressure to obtain 229 g of erythritol crystals.

### 1') Concentration:

The liquid (1.79 kg) was concentrated as such to 1.0 kg. The concentrate had an erythritol concentration of 40.7% and an acetoin concentration of 13.3 ppm. The rate of acetoin removal during the concentration step was 62%.

### 2') Crystallization:

The concentrate obtained in (1') above was slowly cooled in a crystallizer from 57°C to 20°C over a period of 8 hours, and the precipitated crystals were collected by filtration. The wet crystals were washed with 134 g (half the weight of the wet crystals) of cold water at 5°C and then dried under reduced pressure to obtain 229 g of erythritol crystals.

The crystals obtained in (3) and (2') above were compared. Either of them had no irritating chemical smell. The crystals obtained in (3), which had been subjected to ion-exchange resin treatment before concentration, were proved to have more pleasant sweetness close to sucrose than that obtained in (2').

### EXAMPLE 4

### 1) Ion-Exchange Resin Treatment:

The mixture of the mother liquor and the washing obtained in Example 2-(6), which weighed 3.51 kg and had an erythritol concentration of 26.3% and an acetoin concentration of 10.5 ppm, was passed through the same ion exchanger as used in Example 3 at a space velocity of 2 hr⁻¹, and the columns were successively swept with water. At the time when 0.36 ℓ of the liquid was fed to the column, diluted erythritol began to be effused from the mixed bed column, and collection of the effluent was started. The total effluent collected up to the end of the effusion of erythritol amounted to 4.05 kg. The collected effluent had an erythritol concentration of 22.7%.

### 2) Concentration:

The treated liquid was concentrated to 2.02 kg. The concentrate had an erythritol concentration of 45.6% and an acetoin concentration of 5.7 ppm. The rate of acetoin removal during the concentration step was 69%.

### 3) Crystallization:

The concentrate was slowly cooled in a crystallizer from 53°C to 20°C over a period of 8 hours, and the precipitated crystals were collected by filtration. The wet crystals were washed with 274 g (half the weight of the wet crystals)of cold water at 5°C and then dried under reduced pressure to obtain 471 g of erythritol crystals. The resulting crystals were free from an irritating chemical smell and had pleasant sweetness close to sucrose.

### EXAMPLE 5

### 1) Culturing:

In each of three 3 ℓ-volume Erlenmeyer flasks was put 0.6 ℓ of a sterilized seed culture medium containing 300 g/ℓ of crystalline glucose, 20 g/ℓ of yeast extract, and 1.5 g/ℓ of thiamine hydrochloride. The medium was inoculated with Candida lypolytica and shake-cultured at 30°C for 94 hours to prepare a preculture.

In a sterilized 30 ℓ jar fermentor were charged a sterilized aqueous solution containing 6.09 kg of crystalline glucose and a separately sterilized aqueous solution containing 0.34 kg of yeast extract and 25.5 g of thiamine hydrochloride, and 1.70 ℓ of the above obtained preculture was added thereto. After the pH was adjusted to 4.6, the system was subjected to culturing. The system at the commencement of culturing had a volume of 17.0 ℓ, comprising 358 g/ℓ of glucose and 20 g/ℓ of yeast extract, and 1.5 g/ℓ of thiamine hydrochloride. Culturing was carried out at a temperature of 27°C for 120 hours at a stirring speed of 500 rpm and an air feed rate of 1.00 vvm. At the end of the culturing, the culture had a volume of 14.8 ℓ, comprising 215 g/ℓ of erythritol, 16 g/ℓ of residual glucose, 29.0 mg/ℓ of acetoin, and 12.0% by volume of microbial cells.

### 2) Separation of Microbial Cells:

The culture (14.8 ℓ) was separated into wet microbial cells and a supernatant liquor by means of a de Laval type centrifuge. To the wet microbial cells separated was added 2.78 ℓ of water, followed by stirring. The slurry was again separated into wet microbial cells and a supernatant liquor (washing) by means of the same centrifuge. This washing operation was repeated once more under the same conditions. All the washings were combined with the supernatant liquor obtained by the first centrifugal separation, reaching a total of 17.5 ℓ.

### 3) Chromatographic Separation:

Prior to chromatography for desalting, the liquid was concentrated to 8.19 kg. The concentrate had an erythritol concentration of 33.3% and an acetoin concentration of 17.4 ppm. The rate of acetoin removal during the concentration step was 61%.

The concentrate was passed through a column (250 mm in diameter; 1445 mm in packing length) packed with 70.9 ℓ of a strongly acidic cation-exchange resin "Diaion UBK 550, Na type" (produced by Mitsubishi Kasei Corp.) at a space velocity of 0.57 hr⁻¹. After all the liquid was fed, water at 75°C was then supplied. The effluent from the column bottom up to 42.5 ℓ mainly comprised salts present in the concentrate and was discarded. Since the subsequent effluent contained erythritol, collection of the effluent was then started. When the effluent collected amounted to 19.9 ℓ, no erythritol was detected any more, and collection was stopped.

### 4) Ion-Exchange Resin Treatment:

The liquid (19.9 ℓ) was demineralized by passing through an ion exchanger composed of a column packed with 1.5 ℓ of a strongly acidic cation-exchange resin ("Diaion SKIB, H type" produced by Mitsubishi Kasei Corp.), a column packed with 1.5 ℓ of a weakly basic anion-exchange resin ("Diaion WA 30, OH type" produced by Mitsubishi Kasei Corp.), and a mixed bed column packed with a mixture of 0.5 ℓ of a strongly acidic cation-exchange resin ("Diaion SKIB, H type") and 1.0 ℓ of a strongly basic anion-exchange resin ("Diaion PA 408, OH type" produced by Mitsubishi Kasei Corp.), all having a diameter of 50 mm and a packing length of 765 mm, connected in series. After all the liquid was introduced into the columns at a space velocity of 2 hr⁻¹, the columns were swept with water at the same space velocity. At the time point when 2.7 ℓ of the liquid had been fed, diluted erythritol began to effuse from the mixed bed column, at which collection of the effluent was started. The total amount of the effluent collected to the end of effusion of erythritol was 23.9 ℓ (24.9 kg).

### 5) Activated Carbon Treatment:

To the collected effluent was added 28 g of activated carbon, and the mixture was thoroughly stirred and filtered. The separated activated carbon was washed with 85 g of water, and the washing was added to the activated carbon-treated liquid.

The erythritol recovery attained at this point was as high as 97.2% by virtue of the thorough washing of the microbial cells, sweeping of the ion-exchange columns with water, and washing of the activated carbon. Because of the use of a large quantity of water in the washing and column sweeping operations, the amount of the liquid increased to 25.0 kg, while the erythritol concentration decreased to 10.8%.

### 6) Concentration:

The activated carbon-treated liquid was concentrated to 5.37 kg. The concentrate contained 50.2% of erythritol and 3.0 ppm of acetoin. An overall rate of acetoin removal attained by the double-stage concentration was 96%.

### 7) Crystallization:

The concentrate was slowly cooled in a crystallizer from 60°C to 20°C over a period of 8 hours, and the precipitated crystals were collected by filtration. The wet crystals were washed with 770 g of cold water at 5°C and then dried under reduced pressure to recover 1.32 kg of erythritol crystals. The resulting crystals had no irritating smell of chemicals at all.

The crystallizing mother liquor and the washing were combined to amount to 4.82 kg, having an erythritol concentration of 28.3% and an acetoin concentration of 3.4 ppm.

### EXAMPLES 6 TO 12

An erythritol-containing liquid obtained by culturing and purification in the same manner as in Example 2 was concentrated by heating so as to have an erythritol concentration as shown in Table 1 below and an acetoin concentration of not more than 20 ppm. The concentrate (0.8 ℓ) as heated to 85°C was put in a 1.0 ℓ-volume crystallizer equipped with a cooling jacket, and cooled to 15°C at a cooling rate of 0.3°C/min. When the liquid temperature became 3°C lower than the saturation point, erythritol crystals whose particle size is shown in Table 1 were added as seed crystals to the cooling system. The precipitated erythritol crystals were collected by filtration at that temperature.

Any of the thus recovered erythritol crystals was free from an irritating chemical smell. Further, the average particle size and hardness of each crystal were measured, and the results obtained are shown in Table 1. The hardness was obtained by measuring a load required to crush one crystal when applied vertically by means of Tensilon (manufactured by Toyo Sokuki K.K.). An average was obtained from 20 measurement values.

**TABLE 1**

| Example No. | Erythritol/Water Wt. Ratio | Seed Crystals | | | Recovered Erythritol Crystals | | |
|---|---|---|---|---|---|---|---|
| | | Particle Size (µm) | Amount Added (wt%) | Addition Temp. (°C) | Average Particle Size (µm) | Hardness (g/crystal) | Color Tone |
| 6 | 0.89 | 75-105 | 0.010 | 38 | 600 | 320 | good |
| 7 | 0.71 | 75-105 | 0.010 | 31 | 700 | 360 | good |
| 8 | 1.11 | 75-105 | 0.010 | 45 | 500 | 300 | good |
| 9 | 0.89 | 105-150 | 0.030 | 38 | 620 | 350 | good |
| 10 | 0.71 | 53-105 | 0.007 | 31 | 650 | 300 | good |
| 11 | 1.65 | 75-105 | 0.010 | 57 | 780 | 200 | good |
| 12 | 2.28 | 75-105 | 0.010 | 67 | 700 | 180 | good |

According to the present invention, erythritol crystals having very pleasant sweetness close to sucrose and satisfactory crystal properties while being freed of an irritating chemical smell can be prepared with industrial stability.

## Claims

1. A process for preparing erythritol crystals comprising crystallizing an erythritol-containing liquid obtained by fermentation, wherein the acetoin concentration of the erythritol-containing liquid is controlled to 20 ppm by weight or less.

2. A process as claimed in Claim 1, wherein the acetoin concentration of the erythritol-containing liquid is controlled within a range of from 1 to 20 ppm by weight, preferably within a range of from 5 to 15 ppm by weight.

3. A process as claimed in Claim 1 or 2, wherein said erythritol-containing liquid is a liquid obtained by removing microbial cells from a fermentation culture, subjecting the culture liquid to at least one of chromatographic separation, activated carbon treatment, and ion-exchange resin treatment, and then concentrating or diluting the treated liquid.

4. A process as claimed in Claim 1 or 2, wherein said erythritol-containing liquid is a liquid obtained by concentrating or diluting at least a part of a crystallizing mother liquor and/or a washing after crystallizing of erythritol.

5. A process as claimed in Claim 1 or 2, wherein said erythritol-containing liquid is a liquid which is obtained by subjecting at least a part of a crystallizing mother liquor and/or a washing after crystallizing of erythritol to at least one of chromatographic separation, activated carbon treatment, and ion-exchange resin treatment, and then concentrating the treated liquid.

6. A process as claimed in any one of Claims 1 to 5, wherein said erythritol-containing liquid has an erythritol to water weight ratio of from 0.4 to 1.5, preferably of from 0.7 to 1.2.

7. A process as claimed in any one of Claims 1 to 6, wherein said crystallizing is carried out by heating the erythritol-containing liquid to a temperature of from 50° to 90°C, followed by cooling to 20°C or lower.

8. A process as claimed in Claim 7, wherein erythritol seed crystals are added in the course of crystallizing.

9. A process as claimed in Claim 8, wherein said seed crystals are added while the erythritol-containing liquid is between 45°C and 20°C.

10. A process as claimed in Claim 8 or 9, wherein said seed crystals are added in an amount of from 0.005 to 0.5% by weight based on the erythritol in the erythritol-containing liquid.

11. A process as claimed in Claim 10, wherein said seed crystals have a particle size of from 5 to 100 µm.

## Patentansprüche

1. Verfahren zur Herstellung von Erythrit-Kristallen, umfassend die Kristallisation einer Erythrit enthaltenden Flüssigkeit, die durch Fermentation erhalten wurde, wobei die Acetoinkonzentration der Erythrit enthaltenden Flüssigkeit auf 20 Gew.-ppm oder weniger eingestellt wird.

2. Verfahren nach Anspruch 1, wobei die Acetoinkonzentration der Erythrit enthaltenden Flüssigkeit im Bereich von 1 bis 20 Gew.-ppm, vorzugsweise im Bereich von 5 bis 15 Gew.-ppm, eingestellt wird.

3. Verfahren nach Anspruch 1 oder 2, wobei die Erythrit enthaltende Flüssigkeit eine Flüssigkeit ist, die durch Entfernen der Mikrobenzellen von einer Fermentationskultur, mindestens eines der Verfahren: chromatografische Trennung der Kulturflüssigkeit, Behandlung mit Aktivkohle und Ionenaustausch-Behandlung, und dann Konzentrieren oder Verdünnen der behandelten Flüssigkeit erhalten wird.

4. Verfahren nach Anspruch 1 oder 2, wobei die Erythrit enthaltende Flüssigkeit eine Flüssigkeit ist, die durch Konzentrieren oder Verdünnen mindestens eines Teils der Kristallisationsmutterlauge und/oder des Waschwassers nach der Kristallisation des Erythrits erhalten wird.

5. Verfahren nach Anspruch 1 oder 2, wobei die Erythrit enthaltende Flüssigkeit eine Flüssigkeit ist, die dadurch erhalten wird, daß man mindestens einen Teil der Kristallisationsmutterlauge und/oder des Waschwassers nach der Kristallisation des Erythrits mindestens einem der Verfahren: chromatografische Trennung, Behandlung mit Aktivkohle und Ionenaustauschbehandlung, und dann Konzentrieren der behandelten Flüssigkeit unterzieht.

6. Verfahren nach einem der Ansprüche 1 bis 5, wobei die Erythrit enthaltende Flüssigkeit ein Erythrit zu Wasser-Gewichtsverhältnis von 0.4 bis 1.5, vorzugsweise 0.7 bis 1.2, aufweist.

7. Verfahren nach einem der Ansprüche 1 bis 6, wobei die Kristallisation unter Erhitzen der Erythrit enthaltenden Flüssigkeit auf eine Temperatur von 50° bis 90°C, gefolgt von Abkühlen auf 20°C oder weniger durchgeführt wird.

8. Verfahren nach Anspruch 7, wobei im Verlauf der Kristallisation Erythrit-Saatkristalle zugegeben werden.

9. Verfahren nach Anspruch 8, wobei die Saatkristalle zugegeben werden, während die Erythrit enthaltende Flüssigkeit eine Temperatur zwischen 45°C und 20°C aufweist.

10. Verfahren nach Anspruch 8 oder 9, wobei die Saatkristalle in einer Menge von 0.005 bis 0.5 Gew.-%, bezogen auf das Erythrit in der Erythrit enthaltenden Flüssigkeit, zugegeben werden.

11. Verfahren nach Anspruch 10, wobei die Saatkristalle eine Teilchengröße von 5 bis 100 µm aufweisen.

## Revendications

1. Procède de formation de cristaux d'érythritol comprenant la cristallisation d'un liquide contenant de l'érythritol obtenu par fermentation, dans lequel la concentration en acétoïne du liquide contenant l'érythritol est contrôlée à au plus 20 ppm en poids.

2. Procédé selon la revendication 1, dans lequel la concentration en acétoïne du liquide contenant l'érythritol est contrôlée dans une gamme de 1 à 20 ppm en poids et de préférence dans une gamme de 5 à 15 ppm en poids.

3. Procédé selon la revendication 1 ou 2, dans lequel ledit liquide contenant l'érythritol est un liquide obtenu en éliminant les cellules microbiennes d'une culture de fermentation, en soumettant le liquide de culture à au moins une opération parmi la séparation chromatographique, le traitement par du charbon actif et le traitement par une résine échangeuse d'ions, puis en concentrant ou en diluant le liquide traité.

4. Procédé selon la revendication 1 ou 2, dans lequel ledit liquide contenant l'érythritol est un liquide obtenu par concentration ou dilution d'au moins une partie d'une liqueur mère de cristallisation et/ou une solution de lavage après cristallisation de l'érythritol.

5. Procédé selon la revendication 1 ou 2, dans lequel ledit liquide contenant l'érythritol est un liquide qui est obtenu en soumettant au moins une partie d'une liqueur mère de cristallisation et/ou une solution de lavage après cristallisation de l'érythritol à au moins une opération parmi la séparation chromatographique, le traitement par du charbon actif et le traitement par une résine échangeuse d'ions, puis en concentrant le liquide traité.

6. Procédé selon l'une quelconque des revendications 1 à 5, dans lequel ledit liquide contenant de l'érythritol possède un rapport massique de l'érythritol à l'eau de 0,4 à 1,5 et de préférence de 0,7 à 1,2.

7. Procédé selon l'une quelconque des revendications 1 à 6, dans lequel ladite cristallisation est mise en oeuvre en chauffant le liquide contenant l'érythritol à une température de 50 à 90°C, opération suivie d'un refroidissement à au plus 20°C.

8. Procédé selon la revendication 7, dans lequel les cristaux d'inoculation d'érythritol sont ajoutés au cours de la cristallisation.

9. Procédé selon la revendication 8, dans lequel lesdits cristaux d'inoculation sont ajoutés tandis que le liquide contenant l'érythritol a une température comprise entre 45°C et 20°C.

10. Procédé selon la revendication 8 ou 9, dans lequel lesdits cristaux d'inoculation sont ajoutés en une quantité de 0,005 à 0,5% en poids par rapport à l'érythritol dans le liquide contenant l'érythritol.

11. Procédé selon la revendication 10, dans lequel lesdits cristaux d'inoculation ont une dimension de particules de 5 à 100 µm.
